# EUROPÄISCHE PATENTSCHRIFT

(11) **EP 3 106 181 B1**
(45) Veröffentlichungstag und Bekanntmachung des Hinweises auf die Patenterteilung: **23.01.2019**
(21) Anmeldenummer: 16174297.8
(22) Anmeldetag: 14.06.2016
(51) Int. Cl.: A61L 15/26, A61L 15/42, A61L 15/44, A61L 15/46

(54) **VORRICHTUNG ZUR UNTERDRUCKTHERAPIE VON WUNDEN ENTHALTEND SILICONHALTIGEN POLYURETHANSCHAUMSTOFF**
DEVICE FOR THE VACUUM THERAPY OF WOUNDS CONTAINING SILICONE-CONTAINING POLYURETHANE FOAM
DISPOSITIF DE THERAPIE PAR DEPRESSION DE PLAIES COMPRENANT UNE MOUSSE DE POLYURETHANE CONTENANT DU SILICONE

(30) Priorität: 16.06.2015 DE 102015007622
(43) Veröffentlichungstag der Anmeldung: 21.12.2016
(73) Patentinhaber: Paul Hartmann AG, 89522 Heidenheim (DE)
(72) Erfinder: DEIBLER, Martin, 89542 Herbrechtingen (DE)
(74) Vertreter: Ter Meer Steinmeister & Partner

(56) Entgegenhaltungen:
- WO-A1-2009/007018
- WO-A1-2009/126102
- WO-A1-2012/022484
- WO-A1-2012/076439
- WO-A1-2012/167942
- WO-A1-2014/096229
- WO-A1-2015/169637
- "Medical Devices: Guidance document", , 3. Dezember 2009 (2009-12-03), XP055023202, Gefunden im Internet: URL:http://ec.europa.eu/health/medical-dev ices/files/meddev/2_1_3_rev_3-12_2009_en.p df [gefunden am 2012-03-28]
- Dr. Elke Lehmann ET AL: "Abgrenzungsprobleme aus der Sicht des BfArM anhand aktueller Beispiele Bundesinstitut für Arzneimittel und Medizinprodukte", , 26. März 2006 (2006-03-26), XP055312516, Gefunden im Internet: URL:http://www.gd-online.de/german/veranst alt/images2007/E_Lehmann_11.GD_Jahrestagun g_26.03.2007.pdf [gefunden am 2016-10-20]

## Beschreibung

Die Erfindung betrifft eine Vorrichtung zur Unterdrucktherapie von Wunden, umfassend ein Abdeckmaterial zum luftdichten Verschließen der Wunde und der Wundumgebung; ein Mittel, geeignet zur Herstellung von Unterdruck im Wundraum, insbesondere zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind; und einen offenzelligen, quervernetzten silikonhaltigen Polyurethanschaumstoff als Wundauflage, wobei der siliconhaltige Polyurethanschaumstoff erhältlich ist durch Umsetzung einer härtbaren Mischung von Polyisocyanat und Siloxan der Formel (A) wie in den Ansprüchen definiert. Unter einer Wunde wird die Trennung des Zusammenhangs von Geweben der Körperhülle bei Menschen oder Tieren verstanden. Sie kann mit einem Verlust an Substanz verbunden sein.
Die Unterdrucktherapie zur Behandlung von Wunden und entsprechende Vorrichtungen sind seit einiger Zeit im Stand der Technik bekannt.
So beschreibt beispielsweise die WO 93/009727 A1 eine Vorrichtung zur Förderung der Wundheilung durch die Applikation von Unterdruck auf dem die Wunde aufweisenden und die Wunde umgebenden Hautbereich. Die Vorrichtung gemäß WO 93/009727 A1 umfasst eine Vakuumeinrichtung zur Erzeugung des Unterdrucks, eine luftdichte Abdeckung der Wunde, welche mit der Vakuumeinrichtung in einer funktionellen Verbindung steht, sowie eine Wundauflage zur Positionierung an der Wunde innerhalb der luftdichten Abdeckung. Bei der Wundauflage handelt es sich vorzugsweise um einen offenzelligen Polymer-Schaumstoff, beispielsweise um Polyester-Schaum.

WO 2005/123170 A1 offenbart Wundauflagen für die Unterdrucktherapie, mittels derer unerwünschte Substanzen inaktiviert oder aus dem Wundraum entfernt werden sollen und/oder erwünschte Substanzen, welche im Wundraum vorhanden sind, konzentriert werden sollen. Als für den erwünschten Zweck geeignete Bestandteile von Wundauflagen schlägt die WO 2005/123170 eine Vielzahl von zunächst trockenen Polymeren vor, die durch Wasseraufnahme aus dem Wundexsudat ein Gel bilden können.

Die Wundauflagen werden in der Unterdrucktherapie, abhängig vom angelegten Unterdruck, unterschiedlich stark komprimiert. Dadurch kann eine Verengung der für den Abtransport des Wundexsudats nötigen Durchgänge entstehen. Weiterhin kann bei längerer Anwendung in der Unterdrucktherapie eine Verklebung des Schaums der Wundauflage mit dem Wundgrund auftreten. Verklebter Schaum muss beim Wechseln des Verbandes aufwendig entfernt werden, beispielsweise durch Spülen mit Ringerlösung. In den Schaum eingewachsenes Gewebe kann beim Verbandwechsel zu einer Gewebetraumatisierung und damit zur Störung der Wundheilung führen.

Es hat sich ferner gezeigt, dass die viele Polymer-Schaumstoffe nur bedingt für längere Wechselintervalle der Wundauflage geeignet sind. Zunächst ist festzustellen, dass die Durchflussrate innerhalb von drei Tagen nicht selten unerwünscht absinkt und/oder die Saugkraft in Abhängigkeit vom Port unerwünscht abnimmt. Patienten empfinden zudem die üblichen Wundauflagen nicht selten als unangenehm, wenn sie über einen Zeitraum von 3 Tagen ohne Wechsel auf der Wunde verbleiben. Beim Wechsel der Wundauflage nach einem Zeitraum von 3 Tagen oder mehr treten nicht selten unangenehme Gerüche auf. Die Keimdichte ist unerwünscht hoch.

Auch das mögliche Verkleben der Wundauflage mit der Abdeckfolie kann ein Problem darstellen.
Um den vorstehenden Problemen zu begegnen, beschreibt die WO 2011/072840 eine Vorrichtung zur Unterdrucktherapie von Wunden, welche neben einem Abdeckmaterial zum luftdichten Verschließender Wunde und einem zum Unterdruck im Wundraum herstellenden Mittel noch eine Wundauflage, umfasst. Diese Wundauflage ist ein offenzelliger Schaumstoff auf Basis eines quervernetzten Polyorganosiloxans und ist bevorzugt erhältlich durch Umsetzung einer härtbaren Mischung umfassend die Komponenten
(i) ein Polyorganosiloxan enthaltend eine oder mehrere Gruppen mit einer C₂-C₆-Alkenylgruppe, bevorzugt enthaltend eine oder mehrere Vinyl-Gruppen,
(ii) ein Polyorganosiloxan enthaltend eine oder mehrere Si-H-Gruppen,
(iii) ein Treibmittel enthaltend eine oder mehrere OH-Gruppen, und
(iv) einen metallorganischen Katalysator.

WO2009126102 beschreibt Vorrichtung für die Unterdrucktherapie von Wunden, umfassend Wundauflagen, bei denen es sich um mit weichen, hydrophoben Silikon-Gelen imprägnierte offenzellige Polyurethan-Schäume handelt.

Der vorliegenden Erfindung liegt die Aufgabe zugrunde, Alternativen zu bestehenden Lösungen bereitzustellen und die Unterdruckbehandlung von Wunden noch weiter zu verbessern sowie die Nachteile des Standes der Technik zu überwinden. Es sollen mit der vorliegenden Erfindung Vorrichtungen zur Unterdruckbehandlung von Wunden zur Verfügung gestellt werden, mit denen eine Behandlung möglichst wirksam und möglichst schonend durchgeführt werden kann.
Insbesondere soll die vorliegende Erfindung eine Unterdruckbehandlung von Wunden ermöglichen, die beim Patienten einen Wechsel der Wundauflagen in Intervallen von drei Tagen oder länger ermöglicht, werden. Zudem soll eine Wundauflage breitgestellt werden, die einen vorteilhaft möglichst atraumatischen Verbandswechsel ermöglicht, d.h. dass der Verbandswechsel ohne eine weitere Verletzung des Gewebes durchgeführt werden kann.

Beim Wechsel der Wundauflage nach einem Zeitraum von 3 Tagen oder mehr sollen möglichst wenig unangenehme Gerüche auftreten. Die Keimdichte in der ausgewechselten Wundauflage soll niedrig sein. Ferner soll auch bei längeren Wechselintervallen die Bildung von physiologisch bedenklichen Stoffen vermieden werden.
Es soll eine Wundauflage bereitgestellt werden, die ein Verkleben mit der Abdeckfolie weitgehend vermeidet. Die Saugkraft soll nicht in Abhängigkeit vom Port abnehmen.
Die Aufgaben konnten unerwartet durch die Verwendung einer Wundauflage, die einen offenzelligen Schaumstoff auf Basis des offenzelligen, quervernetzten silikonhaltigen Polyurethanschaumstoff als Wundauflage enthält, gelöst werden. Überraschend wurde gefunden, dass eine Vorrichtung zur Unterdrucktherapie umfassend mindestens eine Wundauflage, wobei die Wundauflage den offenzelligen, quervernetzten silikonhaltigen Polyurethanschaumstoff enthält, zu einer vorteilhaften, d.h. sehr wirksamen und sehr schonenden Behandlung von Wunden, besonders geeignet ist. Gegenstand der Erfindung ist daher eine Vorrichtung zur Unterdrucktherapie von Wunden umfassend,
(a) ein Abdeckmaterial zum luftdichten Verschließen der Wunde und der Wundumgebung,
(b) ein Mittel zur Herstellung von Unterdruck im Wundraum, bevorzugt ein Mittel zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind,
(c) einen offenzelligen, quervernetzten silikonhaltigen Polyurethanschaumstoff als Wundauflage, wobei der siliconhaltige Polyurethanschaumstoff erhältlich ist durch Umsetzung einer härtbaren Mischung von Polyisocyanat und Siloxan der Formel (A) wie in den Ansprüchen definiert.

Die neue erfindungsgemäße Vorrichtung zeichnet sich insbesondere dadurch aus, dass sie mindestens eine Wundauflage enthaltend den offenzelligen, quervernetzten silikonhaltigen Polyurethanschaumstoff umfasst. Ein Vorteil des offenzelligen, quervernetzten silikonhaltigen Polyurethanschaumstoffs als Bestandteil der erfindungsgemäßen Vorrichtung besteht darin, dass er eine weiche Wundauflage für die Unterdrucktherapie von Wunden bereitstellt.

Ein weiterer Vorteil des offenzelligen, quervernetzten silikonhaltigen Polyurethanschaumstoffs als Bestandteil der erfindungsgemäßen Vorrichtung besteht darin, dass eine gleichmäßige Druckverteilung auf den Wundgrund gewährleistet werden kann. Durch die Bereitstellung einer weichen Wundauflage und durch die gleichmäßige Druckverteilung kann die Unterdruckbehandlung schonend und besonders wirksam durchgeführt werden.

Weiterhin kann durch den offenzelligen, quervernetzten silikonhaltigen Polyurethanschaumstoff als Wundauflage ein offenzelliger Schaumstoff mit einer größeren Porendicke erreicht werden, was zu einem vorteilhaft höheren aber dennoch konstanten Absaugen des Exsudats führt.

Ein weiterer Vorteil des offenzelligen, quervernetzten silikonhaltigen Polyurethanschaumstoffs als Bestandteil der erfindungsgemäßen Vorrichtung besteht darin, dass ein Verkleben und/oder Verwachsen des Wundgrundes mit der Wundauflage selbst über einen Zeitraum von 3 Tagen oder mehr weitgehend vermieden werden kann. Eine Traumatisierung der Wunde beim Verbandswechsel kann vermieden werden. Dies erhöht die Wirksamkeit der Wundbehandlung.

Ein weiterer Vorteil des offenzelligen, quervernetzten silikonhaltigen Polyurethanschaumstoffs als Bestandteil der erfindungsgemäßen Vorrichtung besteht darin, dass eine Unterdruckbehandlung von Wunden ermöglicht wird, die beim Patienten einen Wechsel der Wundauflagen in Intervallen von drei Tagen oder länger ermöglicht.

Die Wundauflage aus dem offenzelligen, quervernetzten silikonhaltigen Polyurethanschaumstoffs wird im Allgemeinen während der Unterdruckbehandlung gut vertragen und kann zur Förderung der Wundheilung beitragen. Beim Wechsel der Wundauflage nach einem Zeitraum von 3 Tagen oder mehr kann die Keimdichte niedriger als bei herkömmlichen Wundauflagen sein.

Die Bestandteile (a) bis (c) der erfindungsgemäßen Vorrichtung werden nachstehend beschrieben.

Die erfindungsgemäße Vorrichtung umfasst ein Abdeckmaterial (a) zum luftdichten Verschließen der Wunde. Unter "luftdichtem Verschließen" soll hier nicht verstanden werden, dass keinerlei Gasaustausch zwischen dem Wundraum und seiner Umgebung stattfindet. Vielmehr ist mit "luftdichtem Verschließen" in diesem Zusammenhang gemeint, dass unter Berücksichtigung der eingesetzten Unterdruckpumpe der für die Unterdrucktherapie von Wunden nötige Unterdruck aufrechterhalten werden kann. Es können deshalb auch Abdeckmaterialien eingesetzt werden, welche eine geringfügige Gas-Permeabilität aufweisen, so lange der für die Unterdrucktherapie notwendige Unterdruck aufrechterhalten werden kann.

Das luftdichte Abdeckmaterial kann beispielsweise in Form einer aus einem festen Material bestehenden Schale oder in Form einer flexiblen Folie vorliegen. Vorstellbar sind auch Kombinationen von festen Rahmen oder Auflageplatten mit flexiblen Folien.

In einer bevorzugten Ausführungsform der Erfindung umfasst das Abdeckmaterial zum luftdichten Verschließen der Wunde ein wasserunlösliches Polymer oder eine Metallfolie. Das Abdeckmaterial weist bevorzugt eine Dicke von 10 µm bis 10.000 µm, insbesondere von 25 µm bis 100 µm auf.

Als Abdeckmaterial können auch Fertigprodukte eingesetzt werden, welche die vorstehend genannten Eigenschaften aufweisen. Als besonders geeignetes Abdeckmaterial für die erfindungsgemäße Vorrichtung hat sich der Polyurethanfilm der Marke Hydrofilm® (Paul Hartmann AG, Deutschland) oder Visulin® (Paul Hartmann AG, Deutschland) erwiesen.

Das Abdeckmaterial wird üblicherweise in der Wundumgebung oder am Wundrand so befestigt, dass ein luftdichter Wundverschluss gewährleistet ist. Dabei kann es zweckmäßig sein, wenn das Abdeckmaterial vollflächig selbstklebend ausgestattet ist oder einen selbstklebenden Rand aufweist. Alternativ können Befestigung und Abdichtung beispielsweise mit einer Klebefolie, mit einem flüssigen Kleber oder mit einer Abdichtmasse erfolgen.

Die erfindungsgemäße Vorrichtung zur Unterdrucktherapie umfasst ein Mittel (b) zur Herstellung von Unterdruck im Wundraum. In einer bevorzugten Ausführungsform handelt es sich hierbei um ein Mittel zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind.

Der Ausdruck "Unterdruck im Wundraum" bezeichnet im Zusammenhang mit der Erfindung einen innerhalb des Wundverbandes gegenüber dem Umgebungsluftdruck (atmosphärischer Luftdruck) erniedrigten Luftdruck. Unter "innerhalb des Wundverbandes" wird der durch das Abdeckmaterial und die Wunde gebildete Zwischenraum verstanden. "Unterdruck" wird häufig auch als "negativer Druck" bezeichnet.

Die Druckdifferenz zwischen dem Luftdruck innerhalb des Wundverbandes und dem Umgebungsluftdruck wird im Zusammenhang mit der Erfindung in mm Hg (Millimeter-Quecksilbersäule) angegeben, da dies im Bereich der Unterdrucktherapie üblich ist. 1 mm Hg entspricht einem Torr beziehungsweise 133,322 Pa (Pascal). Im Zusammenhang mit der Erfindung wird der Unterdruck, d.h. die Druckdifferenz zwischen dem Luftdruck innerhalb des Wundverbandes und dem Umgebungsluftdruck, als positiver Zahlenwert in mm Hg angegeben.

In einer Ausführungsform der Erfindung handelt es sich bei dem Unterdruck um einen Unterdruck von höchstens 250 mg Hg, bevorzugt höchstens 175 mm Hg. Dieser Unterdruckbereich von höchstens 250 mg Hg, bevorzugt höchstens 175 mm Hg hat sich als für die Wundheilung geeignet erwiesen. In einer bevorzugten Ausführungsform der Erfindung handelt es sich bei dem Unterdruck um einen Unterdruck von mindestens 10 mm Hg und höchstens 150 mm Hg, mehr bevorzugt von mindestens 65 und höchstens 135 mm Hg.

Im Allgemeinen kann es sich bei dem Unterdruck um einen konstanten Unterdruck oder um einen zeitlich variierenden Unterdruck handeln.

Die erfindungsgemäße Vorrichtung kann weiterhin ein Mittel umfassen, so dass der in der Vorrichtung tatsächlich vorhandene Unterdruck überprüfbar und gegebenenfalls einstellbar ist. Das Mittel kann sich im Wundraum oder an einer anderen geeigneten Stelle befinden. So ist es beispielsweise möglich, einen Drucksensor in der Unterdruckleitung zwischen Wundverband und der Unterdruckquelle anzubringen.

Die erfindungsgemäße Vorrichtung zur Unterdrucktherapie von Wunden umfasst weiterhin ein Mittel zur funktionellen Verbindung des Wundraums mit einer außerhalb des Abdeckmaterials befindlichen Unterdruckquelle.

Die funktionelle Verbindung kann beispielsweise mit einer Verbindungsleitung oder mit einem Unterdruck-Anschlussstück hergestellt werden. Unterdruck-Anschlussstücke sind dem Fachmann auch unter der Bezeichnung "Port" bekannt.

Bei einer Ausführungsform handelt es sich bei dem Mittel zur funktionellen Verbindung des Wundraums mit einer außerhalb des Abdeckmaterials befindlichen Unterdruckquelle um mindestens eine Verbindungsleitung. Die mindestens eine Verbindungsleitung kann durch das Abdeckmaterial hindurchgeführt werden.

Alternativ kann die mindestens eine Verbindungsleitung unter dem Rand des Abdeckmaterials durchgeführt werden.

In beiden Fällen ist die Durchtrittsstelle luftdicht abzudichten, so dass der gewünschte Unterdruck im Verband aufrecht erhalten werden kann. Als Abdichtmittel eignet sich beispielsweise eine Klebefolie, eine Klebemasse oder ein Klebestreifen.

Bei der Verbindungsleitung kann es sich beispielsweise um einen Schlauch, um ein Rohr oder um einen anderen Körper mit einem Hohlraum handeln. Ein geeigneter Schlauch ist beispielsweise ein Silicon-Drainageschlauch.

Die Verbindungsleitung verfügt zweckmäßigerweise an dem Ende, welches sich außerhalb des Wundverbandes befindet, über einen Unterdruckadapter, um mit den weiteren Komponenten des Unterdrucksystems verbindbar zu sein.

Die Verbindungsleitung verfügt an dem Ende, welches sich innerhalb des Wundverbandes befindet, eine Öffnung.

In einer weiteren Ausführungsform handelt es sich bei dem Mittel zur funktionellen Verbindung des Wundraums mit einer außerhalb des Abdeckmaterials befindlichen Unterdruckquelle um ein Unterdruck-Anschlussstück (Port), welches auf einer der Innen- oder Außenseiten des Abdeckmaterials befestigt werden kann, wobei das Abdeckmaterial geeignete Öffnungen aufweist. Auch bei dieser Ausführungsform ist auf eine luftdichte Abdichtung entweder der Durchtrittsöffnung (Port innen) oder der Auflagefläche (Port außen) zu achten. Die Abdichtung kann beispielsweise mit einer Klebefolie, mit einer Klebemasse oder mit einem Klebestreifen hergestellt werden. Es ist auch denkbar, dass der Port selbst über entsprechende Befestigungseinrichtungen, wie beispielsweise Klebeflächen, verfügt.

Geeignete Unterdruck-Anschlussstücke sind kommerziell erhältlich. Dabei handelt es sich typischerweise um Unterdruck-Anschlussstücke, welche auf der Außenseite des Abdeckmaterials befestigt werden.

Auch das Unterdruck-Anschlussstück verfügt zweckmäßigerweise über einen Unterdruckadapter, um mit den weiteren Komponenten des Unterdrucksystems verbindbar zu sein.

Neben den vorstehend beschriebenen Bestandteilen (a) und (b) umfasst die erfindungsgemäße Vorrichtung noch Bestandteil (c). Der offenzellige, quervernetzten silikonhaltige Polyurethanschaumstoff (c), welcher in der erfindungsgemäßen Vorrichtung Verwendung findet, wird nachstehend genauer beschrieben.

Als Schaumstoffe werden üblicherweise Werkstoffe mit über die gesamte Masse verteilten Zellen (offen, geschlossen oder beides) verstanden. Werkstoffe weisen üblicherweise somit eine Rohdichte (gemäß DIN EN ISO 845) auf, die niedriger ist als die Dichte der Gerüstsubstanz.

Eine Zelle ist der bei der Herstellung von Schaumstoffen gebildete einzelne Hohlraum, der von Zellwänden und/oder Zellstegen teilweise oder vollständig umschlossen ist.

Eine geschlossene Zelle ist üblicherweise eine Zelle, die vollständig von ihren Wänden umschlossen ist und daher nicht mit anderen Zellen über die Gasphase in Verbindung steht. Eine offene Zelle ist üblicherweise eine Zelle, die über die Gasphase mit anderen Zellen in Verbindung steht. Im Rahmen dieser Anmeldung bedeutet der Begriff offenzellig, dass im Schaumstoff (c) mindestens 60 % offene Zellen, bevorzugt mindestens 90 % offene Zellen, mehr bevorzugt mindestens 98 % offene Zellen, insbesondere im Wesentlichen 100 % offene Zellen, bezogen auf die Gesamtzahl an Zellen, vorhanden sind.

Unter Zellwand wird üblicherweise die die Zelle umschließende Wand verstanden. Die Zellwand kann auch als Zellmembran bezeichnet werden. Als Zellsteg wird üblicherweise der Bereich der Zellwand verstanden, der mehr als zwei Zellen voneinander trennt. Zellstege weisen bevorzugt mindestens das 1,5-fache der Dicke, mehr bevorzugt mindestens das 2-fache der Dicke des übrigen Bereichs der Zellwand auf.

Unter einem retikulierten Schaumstoff wird ein Schaumstoff verstanden, der im Wesentlichen nur Zellstege aufweist.

Allgemein wird unter einem Polyurethan ein Polymer verstanden, welches Urethangruppen, -O-C(O)-NH-, enthält. In Fall des vorliegenden siliconhaltigen Polyurethans enthält das Polyurethan mindestens zwischen zwei Urethangruppen eine Polysiloxan-Verbindung. Unter Polysiloxanen werden synthetische Polymere verstanden, bei denen Siliziumatome über Sauerstoffatome verknüpft sind.

Das siliconhaltige Polyurethan ist quervernetzt. Unter "quervernetzt" wird üblicherweise verstanden, dass lineare Polymerketten über kovalente Bindungen quer (d.h. nicht am Ende der linearen Ketten) miteinander verbunden sind.

Der siliconhaltige Polyurethanschaumstoff (c) ist erhältlich durch Umsetzung einer härtbaren Mischung umfassend die Komponenten:
(i) Polyisocyanat
(ii) Siloxan der Formel (A) 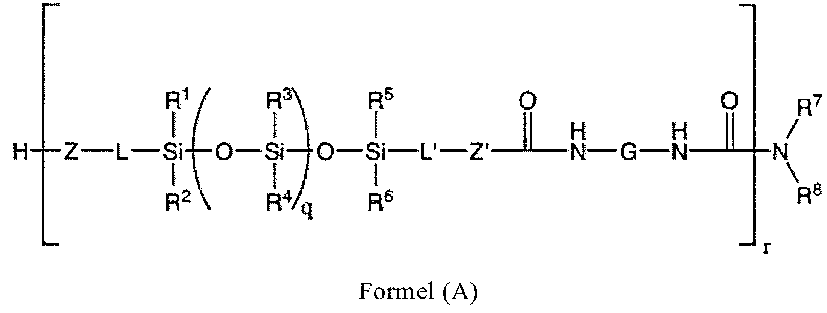
wobei
R¹, R², R³, R⁴, R⁵ und R⁶ unabhängig von einander Wasserstoff, ein substituierter oder ein unsubstituierter aliphatischer Kohlenwasserstoffrest mit 1 bis 8 C-Atomen oder ein substituierter oder ein unsubstituierter aromatischer Kohlenwasserstoffrest mit 6 bis 12 C-Atomen sind,
R⁷ und R⁸ unabhängig von einander Wasserstoff, ein substituierter oder ein unsubstituierter aliphatischer Kohlenwasserstoffrest mit 1 bis 8 C-Atomen oder ein substituierter oder ein unsubstituierter aromatischer Kohlenwasserstoffrest mit 6 bis 12 C-Atomen sind, unter der Voraussetzung, dass mindestens einer der Reste R⁷ und R⁸ mindestens eine Hydroxyl- oder Thiolgruppe aufweist,
Z und Z' unabhängig voneinander Sauerstoff (O) oder Schwefel (S) sind,
L und L' unabhängig von einander ein substituierter oder ein unsubstituierter aliphatischer Kohlenwasserstoffrest mit 1 bis 6 C-Atomen, wobei eines des C-Atome davon durch Sauerstoff (O) ersetzt sein kann, sodass eine Etherbindung vorliegt, oder ein substituierter oder unsubstituierter aromatischer Kohlenwasserstoffrest mit 6 bis 12 C-Atomen ist, welcher ein Heteroatom enthalten können,
G ein substituierter oder ein unsubstituierter aliphatischer Kohlenwasserstoffrest mit 1 bis 6 C-Atomen, oder ein substituierter oder unsubstituierter aromatischer Kohlenwasserstoffrest mit 6 bis 12 C-Atomen ist, welche ein Heteroatom enthalten kann,
q eine ganze Zahl von 1 bis 100 ist,
r eine ganze Zahl von 1 bis 10 ist.

In einer bevorzugten Ausführungsform können die Polyisocyanate (i) Verbindungen mit mindestens 2 bis 8 Isocycanatgruppen, bevorzugt 2 bis 4 Isocycanatgruppen, insbesondere 2 oder 3 Isocycanatgruppen, sein.

Beispiele hierfür sind Diisocyanate wie 1,6-Hexamethylendiisocyant (HDI); 1-Isocyanato-3-isocyanatomethyl-3,5,5-trimethylcyclohexan (Isophorondiisocyanat, IPDI); 4,4'-Diisocyanatodicyclohexyl-methan (H₁₂MDI); 2,4-Toluoldiisocyanat (2,4-TDI); 2,6-Toluoldisiocynat (2,6-TDI); 2,2'-Diphenylmethandisocyanat (2,2'-MDI), 2,4'-Diphenylmethandisocyanat (2,4'-MDI), 4,4'-Diphenylmethandisocyanat (4,4'-MDI), Naphthalin-1,5-diisocyanat (NDI); 1,3-Bis(1-isocyanato-l-methylethyl)-benzol (TMXDI), Triisocyanate wie 4,4',4"-Triphenylmethantrisiocyanat, oder Polyisocyanate wie polymeres MDI (p-MDI) sowie Gemische davon.

Bevorzugt werden die Polyisocyanate (i) in einer Menge von 0,1 bis 150 Gew.-%, bevorzugt von 1 bis 100 Gew.-%, insbesondere 10 bis 50 Gew.-%, eingesetzt; bezogen auf das Gewicht des Siloxans (ii).

Ein aliphatischer Kohlenwasserstoffrest mit 1 bis 8 C-Atomen kann eine Alkylgruppe mit 1 bis 8 C-Atomen, eine Cycloalkygruppe mit 3 bis 8 C-Atomen, eine Alkenylgruppe mit 2 bis 8 C-Atomen, eine Cycloalkenylgruppe mit 3 bis 8 C-Atomen sowie eine Alkinylgruppe mit 2 bis 8 C-Atomen sein.

Beispiele für Alkylgruppen mit 1 bis 8 C-Atomen sind Methyl-, Ethyl-, Propyl-, Isopropyl-, Butyl-, Isobutyl-, tert. Butyl-, Pentyl-, Isopentyl-, neo-Pentyl-, Hexyl-, Isohexyl-, Heptyl- und Oktylgruppen.

Beispiele für Cycloalkylgruppen mit 3 bis 8 C-Atomen sind Cyclopropyl-, Cyclobutyl-, Cyclopentyl-, Cyclohexyl-, Cyclohepyl- und Cyclooctylgruppen.

Beispiele für Alkenylgruppen mit 2 bis 8 C-Atomen sind Vinyl-, Allyl-, 3-Methyl-2-Butenyl-Gruppen.

Beispiele für Cycloalkenylgruppen mit 3 bis 8 C-Atomen sind Cyclopropenyl-, Cyclopentenyl-, Cyclohexenyl- und Cyclooctenylgruppen.

Beispiele für Alkinylgruppen mit 2 bis 8 C-Atomen sind Ethinyl-, 1-Propinyl- und 2-Propinylgruppen.

Beispiele für einen aromatischen Kohlenwasserstoffrest mit 6 bis 12 C-Atomen sind Benzol-, Indan-, Naphthalin- und 1,2,3,4- Tetrahydronaphthalingruppen.

Der aliphatische Kohlenwasserstoffrest mit 1 bis 8 C-Atomen und der aromatische Kohlenwasserstoffrest mit 6 bis 12 C-Atomen können substituiert sein und einen oder mehrere Substituenten tragen.

Beispiele für Substituenten sind Halogenide, Nitro, Cyano, Carbonsäuren, Carbonsäureester, Carbonsäureamide, Hydroxy, optional geschütztes Hydroxy, Amin, optional geschützes Amin, Monoalkylamin, optional geschützes Monalkylamin, Dialkylamin, Alkylgruppen mit 1 bis 6 C-Atomen, Alkoxygruppen mit 1 bis 6 C-Atomen, Aryloxygruppen mit 6 bis 12 C-Atomen.

Schutzgruppen für Amine sind beispielsweise Boc (*tert*-Butyloxycarbonyl), Z oder Cbz (Benzyloxycarbonyl), Benzyl, Benzhydryl und Fmoc (Fluorenylmethylenoxycarbonyl).

Schutzgruppen für Hydroxygruppen sind beispielsweise Ester, wie Benzoesäure- oder Pivalinsäureester, und Silylether wie Trimethylsilylether, Triethylsilylether, tert-Butyldimethylsilylether und tert-Butyldiphenylsilylether.

In einer bevorzugten Ausführungsform sind jeweils R¹ und R², R³ und R4 sowie R⁵ und R⁶ identische Reste, bevorzugt aliphatische Reste mit 1 bis 6 C-Atomen. Besonders bevorzugt sind R¹, R², R³, R⁴, R⁵ und R⁶ identische Reste, mehr bevorzugt aliphatische Reste mit 1 bis 6 C-Atomen, noch mehr bevorzugt aliphatische Reste mit 1 oder 2 C-Atomen, insbesondere Methyl.

Alternativ besonders bevorzugt sind R¹, R², R³, R⁴, R⁵ und R⁶ identische Reste, mehr bevorzugt aromatische Reste mit 6 bis 12 C-Atomen, insbesondere Phenyl.

In einer bevorzugten Ausführungsform können R⁷ und R⁸ identische Reste sein und jeweils eine Hydroxygruppe aufweisen. Besonders bevorzugt sind R⁷ und R⁸ eine Hydroxylalkylgruppe mit 1 bis 4 C-Atomen, insbesondere Hydroxyethyl.

Z und Z' können vorzugsweise identisch sein. Besonders bevorzugt sind Z und Z' jeweils Sauerstoff (O).

In einer bevorzugten Ausführungsform sind L und L' unabhängig voneinander ein substituierter oder ein unsubstituierter aliphatischer Kohlenwasserstoffrest mit 1 bis 6 C-Atomen, wobei eines des C-Atome davon durch Sauerstoff (O) ersetzt sein kann, sodass eine Etherbindung vorliegt.

Weiter ist bevorzugt, dass L und L' identisch sind.

Besonders bevorzugt sind L und L' jeweils ein unsubstituierter aliphatischer Kohlenwasserstoffrest mit 1 bis 4 C-Atomen, wobei eines der C-Atome davon durch Sauerstoff (O) ersetzt sein kann, sodass eine Etherbindung vorliegt. Insbesondere sind L und L' -CH₂- (Methylen) oder -CH₂-O-CH₂-CH₂-(Ethoxymethylen), im speziellen Methylen.

In einer bevorzugten Ausführungsform ist G ein substituierter aromatischer Kohlenwasserstoffrest mit 6 bis 13 C-Atomen. Beispiele für G sind folgende: Phenylen, 2,4-Toluolylen, 2,6-Toluolylen, 1,3-bis-(2-Propylen)-phenylen, Methylen-bis-phenylen, mehr bevorzugt 2,4-Toluolylen, 2,6-Toluolylen, insbesondere 2,4-Toluolylen.

q kann vorzugsweise eine ganze Zahl von 1 bis 100 sein, mehr bevorzugt eine ganze Zahl von 10 bis 75, noch mehr bevorzugt eine ganze Zahl von 20 bis 55, insbesondere ein ganze Zahl 25 bis 40.

r kann vorzugsweise eine ganze Zahl von 1 bis 10 sein, mehr bevorzugt eine ganze Zahl von 1 bis 5, noch mehr bevorzugt 1, 2 oder 3, insbesondere 2.

Das Siloxan der Formel (A) wird vorzugsweise durch die Umsetzung von
- linearem Siloxan (a1) mit der Formel (A1) 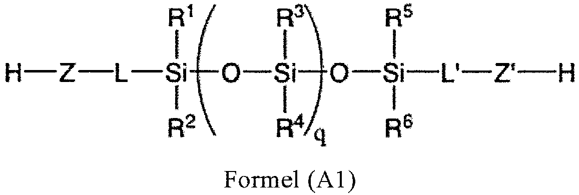 wobei R¹, R², R³, R⁴, R⁵, R6, Z, Z', L, L' und q wie vorstehend definiert sind.
- Diisocyant (a2)
- Amin (a3) mit der Formel (A3) 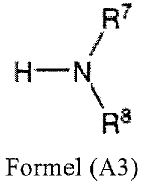 wobei R⁷ und R⁸ wie vorstehend definiert sind.

In einer bevorzugten Ausführungsform sind bei dem linearen Siloxan mit Formel (A1)
R¹, R², R³, R⁴, R⁵, R6 jeweils Methylgruppen,
Z, Z' jeweils Sauerstoff (O),
L, L' jeweils eine Methylengruppe, und
q eine ganze Zahl von 25 bis 40.

Vorzugsweise sind die Diisocyanate (a2) ausgewählt aus 1,6-Hexamethylendiisocyant (HDI); 1-Isocyanato-3-isocyanatomethyl-3,5,5-trimethylcyclohexan (Isophorondiisocyanat, IPDI); 4,4'-Diisocyanatodicyclohexyl-methan (H₁₂MDI); 2,4-Toluoldiisocyanat (2,4-TDI); 2,6-Toluoldisiocynat (2,6-TDI); 2,2'-Diphenylmethandisocyanat (2,2'-MDI), 2,4'-Diphenylmethandisocyanat (2,4'-MDI), 4,4'-Diphenylmethandisocyanat (4,4'-MDI), Naphthalin-1,5-diisocyanat (NDI).

Das Gewichtsverhältnis von Diisocyanat (a2) zu linearem Siloxan (a1) mit der Formel (A1) kann 1 : 1000 bis 4 : 10, bevorzugt von 1 : 500 bis 1 : 5, insbesondere vom 1 : 100 bis 1 : 10 betragen.

Die Amine (a3) sind bevorzugt Dialkylamine, wobei die Alkylgruppen unabhängig 1 bis 6 C-Atome aufweisen, und wobei mindestens eine der Alkylgruppen eine Hydroxylgruppe aufweist. Bevorzugt sind N-Methylethanolamin, Diethanolamin und Bis(2-hydroxypropyl)amin, mehr bevorzugt Diethanolamin und Bis(2-hydroxypropyl)amin, insbesondere Diethanolamin.

Das Gewichtsverhältnis von Amin (a3) zu linearem Siloxan (a1) mit der Formel (A1) kann 1 : 1000 bis 3 : 10, bevorzugt von 1 : 500 bis 1 : 10, insbesondere vom 1 : 200 bis 1 : 20 betragen.

Die Umsetzung der Komponenten (a1), (a2) und (a3) zum Siloxan mit der Formel (A) kann bevorzugt ohne Lösungsmittel durchgeführt werden.

Alternativ bevorzugt kann die Umsetzung auch in einem organischen Lösungsmittel durchgeführt werden. Geeignete Lösungsmittel sind beispielsweise Ether wie Diethylether, Dioxan oder Tetrahydrofuran, Ester wie Ethyl- oder Butylacetat, Ketone, wie Aceton oder Methylethylketon, aliphatische Kohlenwasserstoffe wie Pentan, Hexan, Heptan, Cyclopentan oder Cyclohexan, halogenierte Kohlenwasserstoffe, wie Methylenchlorid oder Chloroform, Acetonitril, DMF und aromatische Kohlenwasserstoff wie Benzol, Toluol oder Xylol.

Bei Einsatz eines organischen Lösungsmittels kann dieses von 10 bis 500 Gew.-%,vorzugsweise 20 bis 400 Gew.-%, insbesondere 35 bis 250 Gew.-% eingesetzt werden, bezogen auf das lineare Siloxan (a1) mit der Formel (A1).

Die oben erwähnte Umsetzung zum Siloxan der Formel (A) kann bevorzugt in der Gegenwart eines Katalysators durchgeführt werden. Der Katalysator ist bevorzugt eine metallorganische Verbindung, wobei das Metall aus Eisen, Kupfer, Zink, Zinn, Bismut und Zirkonium ausgewählt ist. Mehr bevorzugt handelt es sich bei dem Metall um Zinn, Bismut und Zirkonium, insbesondere Bismut. Ganz besonders bevorzugt ist Bi-(III)-neodecanoat.

Der Katalysator kann in einer Menge von 1 bis 1000 Gew.-ppm, bevorzugt von 5 bis 500 Gew.-ppm, insbesondere von 10 bis 200 Gew.-ppm bezogen auf das Gesamtgewicht der Komponenten (a1), (a2) und (a3) eingesetzt werden.

Bevorzugt wird die Umsetzung zur Formel (A) in zwei Schritten durchgeführt:
1) Reaktion des Siloxans (a1) mit der Formel (A1) mit einem Diisocyanat (a2) und
2) Umsetzung der nach 1) erhaltenen Reaktionsmischung mit Amin (a3).

Schritt 1) kann bevorzugt in Gegenwart von Lösungsmittel und/oder Katalysator durchgeführt werden.

Schritt 1) kann bei Temperaturen von 0 bis 100 °C, bevorzugt 10 bis 80 °C, mehr bevorzugt von 30 bis 60 °C durchgeführt werden.

Die Reaktionszeit von Schritt 1) kann 1 bis 120 Minuten, bevorzugt 5 bis 90 Minuten, besonders bevorzugt 15 bis 60 Minuten betragen.

In Schritt 2) wird dem Reaktionsgemisch von Schritt 1) das Amin (a3) zugegeben. Die Temperatur und Reaktionszeit von Schritt 2) entspricht im Wesentlichen der von Schritt 1). Nach Beendigung der Reaktion kann gegebenenfalls das Lösungsmittel entfernt werden, bevorzugt bei einer Temperatur von 20 bis 40 °C und einem (reduzierten) Druck von 1 bis 100 hPa.

In einer bevorzugten Aufführungsform kann die härtbare Mischung, aus welcher der siliconhaltige Polyurethanschaumstoff erhältlich ist, noch weitere Komponenten enthalten. Beispiele für solche Komponenten sind (iii) Katalysatoren und (iv)Treibmittel.

Als Katalysatoren (iii) können Verbindungen eingesetzt werden, welche die Reaktion der Komponente (i), Polyisocyanat, mit der Komponente (ii), Siloxan, mit der Formel (A) beschleunigen. In Frage kommen beispielsweise tertiäre Amine und/oder organische Metallverbindungen, wie Zink, Zinn, Bismut und Zirkoniumverbindungen, insbesondere organische Zinnverbindungen. Beispielsweise können als Katalysatoren folgende Verbindungen eingesetzt werden: Triethylendiamin, Aminoalkyl- und/oder Aminophenylimidazole und/oder Zinn-(II)-salze von organischen Carbonsäuren. Katalysatoren werden im Allgemeinen in einer Menge von 0,1 bis 5 Gew.-% bezogen auf das Gesamtgewicht der Komponenten (i) und (ii) eingesetzt.

Die Treibmittel (iv) können physikalische und/oder chemische Treibmittel sein. Beispiele für physikalische Treibmittel sind (cyclo)aliphatische Kohlenwasserstoffe, vorzugsweise solche mit 4 bis 8, besonders bevorzugt 4 bis 6 und insbesondere 5 Kohlenstoffatomen, teilhalogenierte Kohlenwasserstoffe oder Ether, Ketone oder Acetate.

Ein chemisches Treibmittel ist ein Treibmittel, das vorzugsweise eine oder mehrere OH-Gruppen enthält. Beispiele für Verbindungen, die eine oder mehrere OH-Gruppen enthalten, sind Wasser (H-OH) und Alkohole mit 1 bis 12 C-Atomen. Besonders bevorzugt ist Wasser.

Es hat sich zu Erreichung der nachstehend beschriebenen physikalischen Eigenschaften gezeigt, dass es vorteilhaft ist, wenn die Wassermenge in der härtbaren Mischung begrenzt ist. Bevorzugt weist die härtbare Mischung einen Wassergehalt von 0,0001 bis 5 Gew.-%, mehr bevorzugt von 0,001 bis 3,0 Gew.-%, noch mehr bevorzugt von 0,01 Gew.-% bis 1,0 Gew.-%, auf. Der Wassergehalt wird hierbei nach der coulometrischen Karl-Fischer-Methode bestimmt. Üblicherweise ergibt sich der Wassergehalt nicht durch Zugabe von Wasser, sondern durch den Restwassergehalt der Komponenten der härtbaren Mischung.

Die Umsetzung erfolgt gegebenenfalls in Anwesenheit von (v) Hilfs- und/oder Zusatzstoffen, wie z. B. Füllstoffen, Zellreglern, Zellöffnern, oberflächenaktiven Verbindungen und/oder Stabilisatoren gegen oxidativen, thermischen oder mikrobiellen Abbau oder Alterung. Die Hilfs- und/oder Zusatzstoffe können in einer Menge von 0,1 bis 30 Gew.-%, bevorzugt 0,5 bis 20 Gew.-%, mehr bevorzugt 1 bis 15 Gew.-% vorliegen, bezogen auf Polysiloxan mit der Formel (A).

Es hat sich weiterhin gezeigt, dass vorstehend genannte Aufgaben unerwartet vorteilhaft gelöst werden können, wenn der offenzellige, quervernetzte siliconhaltige Polyurethanschaumstoff Substanzen mit antimikrobieller Wirkung (vii) enthält.

Eine mögliche Substanz mit antimikrobieller Wirkung ist Silber und kann in Form von Silberionen oder in Form von atomarem Silber in dem offenzelligen, quervernetzten siliconhaltigen Polyurethanschaumstoff enthalten sein. Bevorzugt wird nach der Herstellung des offenzelligen, quervernetzten siliconhaltigen Polyurethanschaumstoffs eine Silberbeschichtung aufgebracht. Alternativ kann das Silber bereits in die härtbare Zusammensetzung miteingebracht werden. Bevorzugt enthält der offenzellige, quervernetzte siliconhaltige Polyurethanschaumstoff 0,000001 bis 0,1 Gew.-%, mehr bevorzugt 0,0001 bis 0,01 Gew.-% Silber, bezogen auf das Gesamtgewicht des offenzelligen, quervernetzten siliconhaltigen Polyurethanschaumstoffs.

Des Weiteren kann der offenzellige, quervernetzte siliconhaltige Polyurethanschaumstoff auch organische Substanzen mit antimikrobieller Wirkung enthalten. Bei der Substanz mit antimikrobieller Wirkung kann es sich beispielsweise um Substanzen mit Amino- oder Iminogruppen handeln. Weiterhin kann es sich bei der Substanz mit antimikrobieller Wirkung um antimikrobiell wirksame Metallkationen handeln, insbesondere um Silberkationen, beispielsweise um einen Komplex von 1-Vinyl-2-Pyrrolidone mit Silberkationen. Besonders geeignete Substanzen mit antimikrobieller Wirkung sind weiterhin Biguanid-Derivate, wie Chlorhexidin, oder Polybiguanide, wie Polyethylenbiguanid (PEB), Polytetramethylenbiguanid (PTMB) oder Polyethylenhexamethylenbiguanid (PEHMB). Ein besonders bevorzugtes Polybiguanid ist Polyhexamethylenbiguanid (PHMB bzw. Polyhexanid). Weitere geeignete Substanzen mit antimikrobieller Wirkung sind Polyguanidine, wie zum Beispiel Polyhexamethylenguanidine (PHMG), *N*-Octyl-1-[10-(4-Octyliminopyridin-1-yl)decyl]pyridin-4-imin (Octenidin), quartäre Ammoniumverbindungen, wie zum Beispiel Benzalkoniumchlorid oder Cetylpyridiniumchlorid, Triazine, wie zum Beispiel 1-(3-Chloroallyl)-3,5,7-triaza-1-azonia-adamantan-Chlorid oder die Ammoniumverbindung Taurolidin.

Bevorzugt wird der offenzellige, quervernetzte siliconhaltige Polyurethanschaumstoff mit den vorstehend erläuterten Substanzen mit antimikrobieller Wirkung imprägniert oder beschichtet.

Substanzen mit antimikrobieller Wirkung sind üblicherweise in dem offenzelligen, quervernetzten siliconhaltigen Polyurethanschaumstoff in einer Menge von 0 bis 30 Gew.-%, bevorzugt von 0,1 bis 15 Gew.-%, bezogen auf das Gesamtgewicht des offenzelligen, quervernetzten siliconhaltigen Polyurethanschaumstoffs, enthalten.

Um den offenzelligen, quervernetzten siliconhaltigen Polyurethanschaumstoff herzustellen, werden die oben beschriebenen Komponenten in irgendeiner erwünschten Reihenfolge vermengt. Es ist bevorzugt, dass zwei Teilmischung A und B miteinander vermischt werden. Weiterhin ist besonderes bevorzugt, dass die Teilmischung, welche die Komponente (i) enthält, weder die Komponente (ii) noch gegebenenfalls Treibmittel wie z.B. Wasser enthält. Die Vermischung kann durch übliche Mischgeräte erfolgen, beispielsweise mit einem statischen Mischer oder einem dynamischen Mischer.

Die Umsetzung der Komponenten (i) bis (iv) der härtbaren Zusammensetzung erfolgt bevorzugt bei 20 bis 25 °C, insbesondere bei 23 °C.

Die vorstehend erläuterten Komponenten (i) bis (iv) werden bevorzugt so gewählt, dass ein offenzelliger Schaumstoff auf Basis des offenzelligen, quervernetzten siliconhaltigen Polyurethans, insbesondere ein Schaumstoff mit den nachfolgend beschriebenen physikalischen Eigenschaften, erhalten wird.

Es hat sich gezeigt, dass vorstehend genannte Aufgaben unerwartet vorteilhaft gelöst werden können, wenn der offenzellige, quervernetzte siliconhaltige Polyurethanschaumstoff eine spezielle Zugfestigkeit, eine spezielle Bruchdehnung und einer spezielle Härte aufweist. In einer bevorzugten Ausführungsform weist der offenzellige, quervernetzte siliconhaltige Polyurethanschaumstoff eine Zugfestigkeit von 100 kPa bis 100 MPa, mehr bevorzugt 500 kPa bis 50 MPa, noch mehr bevorzugt 800 kPa bis 10 MPa, gemessen gemäß DIN 53571, auf. Ferner weist der offenzellige, quervernetzte siliconhaltige Polyurethanschaumstoff bevorzugt eine Bruchdehnung von 100 % bis 350 %, mehr bevorzugt von 160 % bis 320 %, noch mehr bevorzugt von 180 % bis 300 %, gemessen gemäß DIN 53571, auf. Zudem weist der offenzellige, quervernetzte siliconhaltige Polyurethanschaumstoff bevorzugt eine Härte von 20 bis 70 Shore A, mehr bevorzugt von 30 bis 60 Shore A, noch mehr bevorzugt von 40 bis 50 Shore A, gemessen gemäß DIN 53505, auf.

Es hat sich ferner gezeigt, dass vorstehend genannte Aufgaben unerwartet vorteilhaft gelöst werden können, wenn der offenzellige, quervernetzte siliconhaltige Polyurethanschaumstoff eine spezielle Luftdurchlässigkeit aufweist. In einer bevorzugten Ausführungsform weist der Schaumstoff (c) eine Luftdurchlässigkeit von 1000 bis 8000 l/(m²sec), mehr bevorzugt von 2000 bis 7500 l/(m²sec), noch mehr bevorzugt von 2500 bis 7000 l/(m²sec), insbesondere von 3000 bis 6500 l/(m²sec), gemessen gemäß DIN EN ISO 9237, auf.

Es hat sich weiterhin gezeigt, dass vorstehend genannte Aufgaben unerwartet vorteilhaft gelöst werden können, wenn der offenzellige, quervernetzte siliconhaltige Polyurethanschaumstoff viskoelastisches Verhalten zeigt. Hierunter wird verstanden, dass das Verhalten des offenzelligen, quervernetzten siliconhaltigen Polyurethanschaumstoffs auf Beanspruchung so aussieht, wie die Kombination aus einem elastischen Feststoff und einer viskosen Flüssigkeit. Das viskoelastische Verhalten kann durch einen Torsionsschwingungsversuch gemäß DIN 53445 charakterisiert werden. Es ist bevorzugt, dass der Schaumstoff bei der Bestimmung gemäß DIN 53445 bei 23 °C einen mechanischen Verlustfaktor von 0,1 bis 1,0, mehr bevorzugt von 0,15 bis 0,8, noch mehr bevorzugt von 0,2 bis 0,6 aufweist.

Es hat sich weiterhin gezeigt, dass vorstehend genannte Aufgaben unerwartet vorteilhaft gelöst werden können, wenn der offenzellige, quervernetzte siliconhaltige Polyurethanschaumstoff eine Rohdichte zwischen 5 und 100 kg/m³, mehr bevorzugt wischen 10 und 60 kg/m³, insbesondere zwischen 15 und 35 kg/m³ aufweist, gemessen gemäß DIN EN ISO 845. Als besonders vorteilhaft hat sich ein offenzelliger, quervernetzter siliconhaltiger Polyurethanschaumstoff mit einer Rohdichte im Bereich zwischen 24 und 28 kg/m³ erwiesen.

Es hat sich weiterhin gezeigt, dass vorstehend genannte Aufgaben unerwartet vorteilhaft gelöst werden können, wenn der offenzellige, quervernetzte siliconhaltige Polyurethanschaumstoff Silber in Form von Silberionen oder in Form von atomarem Silber enthält. Bevorzugt wird nach der Herstellung des offenzelligen, quervernetzten siliconhaltigen Polyurethanschaumstoffs eine Silberbeschichtung aufgebracht. Alternativ kann das Silber bereits in die härtbare Zusammensetzung mit eingebracht werden. Bevorzugt enthält der offenzellige, quervernetzte siliconhaltige Polyurethanschaumstoff 0,000001 bis 0,1 Gew.-%, mehr bevorzugt 0,0001 bis 0,01 Gew.-% Silber, bezogen auf das Gesamtgewicht des offenzelligen, quervernetzten siliconhaltigen Polyurethanschaumstoffs.

In einer bevorzugten Ausführungsform wird der offenzellige, quervernetzte siliconhaltige Polyurethanschaumstoff in trockenem Zustand eingesetzt. Bevorzugt ist somit der Schaumstoff z.B. nicht mit einer Aktivierungslösung (wie z.B. Ringerlösung) getränkt.

In einer bevorzugten Ausführungsform der Erfindung weist der offenzellige, quervernetzte siliconhaltige Polyurethanschaumstoff eine Dicke von 1 bis 50 mm, insbesondere von 15 mm bis 30 mm auf.

In einer bevorzugten Ausführungsform der Erfindung umfasst die Vorrichtung zur Unterdrucktherapie von Wunden mindestens eine Wundkontaktschicht zum Einbringen zwischen den offenzelligen, quervernetzten siliconhaltigen Polyurethanschaumstoff und die Wundoberfläche. Die zusätzliche Wundkontaktschicht kann mit dem offenzelligen, quervernetzten siliconhaltigen Polyurethanschaumstoff haftend oder nicht haftend verbunden sein.

Als Wundkontaktschicht kommt grundsätzlich jede aus dem Stand der Technik bekannte Wundkontaktschicht in Frage, solange einerseits ein Durchtritt von Wundexsudat gewährleistet ist und das Material anderseits keine Neigung zum Verwachsen oder Verkleben mit dem Wundgewebe aufweist.

Beispiele für geeignete Wundkontaktschichten sind in den Deutschen Patentanmeldungen DE 10 2008 062 472, DE 10 2008 031 183 und DE 10 2008 031 182 beschrieben.

Ebenso kann die Wundkontaktschicht eine durchlässige Non-woven-Schicht oder einen Gittertüll umfassen. Die durchlässige Non-woven-Schicht oder der Tüll besteht vorzugsweise aus einem hydrophoben Material, beispielsweise Polyester. Der Tüll kann weiterhin mit einer Salbe und/oder einer Silberbeschichtung ausgestattet sein. Besonders geeignete Wundkontaktschichten sind Salbenkompressen der Marke Hydrotüll® und Atrauman® (Paul Hartmann AG, Deutschland).

In einer weiteren bevorzugten Ausführungsform umfasst die Vorrichtung zur Unterdrucktherapie von Wunden mindestens eine zusätzliche Druckverteilungsschicht zwischen dem offenzelligen, quervernetzten siliconhaltigen Polyurethanschaumstoff und dem Abdeckmaterial (a).

Der Vorteil einer zusätzlichen Druckverteilungsschicht kann darin bestehen, dass der durch den Verband auf den Wundgrund ausgeübte Druck durch die Verwendung der Druckverteilungsschicht noch gleichmäßiger verteilbar ist. Weiterhin kann die Druckverteilungsschicht zusätzliches Wundexsudat speichern. Die zusätzliche Druckverteilungsschicht kann aus einem offenzelligen oder halboffenzelligen Schaumstoff, einem Abstandsgewirke, aus einer Textilschicht, aus einem strukturiertem Gel oder aus einer durchlässigen Non-woven-Schicht bestehen. Geeignete Textilschichten sind unter anderem ES-Kompressen oder Gittertülls.

Die zusätzliche Druckverteilungsschicht kann so ausgebildet sein, dass Flüssigkeit, wie Wundexsudat, durch sie hindurchgeleitet wird. Dazu kann die Druckverteilungsschicht geeignete Kanäle oder Öffnungen enthalten oder aus einem für Flüssigkeiten durchlässigen Material bestehen.

Weiterhin stellt die Erfindung ein gebrauchsfertiges Set zur Unterdruck-Wundbehandlung bereit, umfassend die erfindungsgemäße Vorrichtung, wobei der offenzellige, quervernetzte siliconhaltige Polyurethanschaumstoff als Wundauflage geeignet ist und gebrauchsfertig abgepackt vorliegt.

Der vom Set umfasste abgepackte offenzellige, quervernetzte siliconhaltige Polyurethanschaumstoff kann hergestellt werden, indem der trockene offenzellige, quervernetzte siliconhaltige Polyurethanschaumstoff feuchtigkeitsdicht abgepackt wird. Vorzugsweise liegt der gebrauchsfertig abgepackte offenzellige, quervernetzte siliconhaltige Polyurethanschaumstoff in steriler Form vor. Idealerweise liegt der offenzellige, quervernetzte siliconhaltige Polyurethanschaumstoff in steriler, einzeln eingesiegelter, gebrauchsfertiger Packung vor. Die Sterilisation kann durch eine Gegendruckdampfsterilisation oder durch andere, dem Fachmann als geeignet bekannte Sterilisationsmethoden erfolgen.

Das Set kann weitere optionale Bestandteile wie beispielsweise eine oder mehrere Wundkontaktschichten, eine oder mehrere zusätzliche Druckverteilungsschichten, Klebemittel zum Fixieren des Verbands, Abdichtmittel zur Herstellung einer luftundurchlässigen Abdichtung des Verbandes, Drucksensoren, Verbindungselemente für Drucksensoren, zusätzliche Schläuche, Verbindungsstücke für Schläuche, Desinfektionsmittel, Hautpflegemittel, pharmazeutische Zubereitungen oder eine Gebrauchsanweisung enthalten. Bevorzugt enthält das erfindungsgemäße Set ferner Schere, Tupfer und/oder Pinzette, insbesondere in steriler Form.

Das Set kann sowohl die mindestens eine Wundkontaktschicht, als auch die mindestens eine zusätzliche Druckverteilungsschicht umfassen.

Vorzugsweise umfasst das Set weiterhin eine gebrauchsfertige Unterdruckeinheit. Die Unterdruckeinheit kann Komponenten wie beispielsweise eine Pumpe, ein oder mehrere Flüssigkeitsbehälter, eine Steuereinheit, eine Stromversorgung, elektrische Anschlussmittel und Schläuche enthalten. Die Unterdruckeinheit kann auch eine Vorrichtung zur funktionellen Verbindung des Unterdruckverbandes mit einer vorhandenen stationären Unterdruckquelle enthalten. Vorzugsweise werden alle Komponenten, bei denen dies aus medizinischer Sicht notwendig ist, steril abgepackt zur Verfügung gestellt. Der Vorteil des gebrauchsfertigen Sets besteht darin, dass der Unterdruckverband schnell, standardisiert und unkompliziert angelegt werden kann. Ein weiterer Vorteil besteht darin, dass alle im Wundbereich eingesetzten Bestandteile des Sets bereits sterilisiert zur Verfügung gestellt werden können.

Beschrieben wird die Verwendung des vorstehend erläuterten offenzelligen Schaumstoffs auf Basis des offenzelligen, quervernetzten siliconhaltigen Polyurethanschaumstoffs als Wundauflage in der Unterdrucktherapie von Wunden. Beschrieben wird somit auch ein offenzelliger Schaumstoff auf Basis des offenzelligen, quervernetzten siliconhaltigen Polyurethanschaumstoffs zur Anwendung in der Unterdrucktherapie von Wunden. Beschrieben wird somit auch die Verwendung eines offenzelligen Schaumstoffs auf Basis des offenzelligen, quervernetzten siliconhaltigen Polyurethanschaumstoffs in einer Vorrichtung zur Unterdrucktherapie von Wunden, insbesondere als Wundauflage.

Besondere Vorteile durch die erfindungsgemäße Vorrichtung, durch das erfindungsgemäße Set oder die erfindungsgemäße Verwendung, beziehungsweise Anwendung, ergeben sich, wenn es sich bei den Wunden um Brandwunden, um durch mechanische Traumatisierung entstandene Wunden, um durch Einwirkung von Chemikalien entstandene Wunden, um durch Stoffwechselstörung verursachte Wunden, um durch Durchblutungsstörungen verursachte Wunden oder um durch ein Druckgeschwür verursachte Wunden handelt.

In einer weiteren bevorzugten Ausführungsform wird ein offenzelliger Schaumstoff auf Basis des offenzelligen, quervernetzten siliconhaltigen Polyurethanschaumstoffs zur Anwendung in der Unterdrucktherapie bei der Behandlung einer durch eine Hauttransplantation entstandenen Wunde bereitgestellt. Die Anwendung umfasst die Behandlung von durch Spalthaut-Transplantationen und von durch Vollhaut-Transplantationen entstandenen Wunden mittels Unterdrucktherapie. Vorteilhafte Wirkungen ergeben sich durch die weiche Struktur des offenzelligen, quervernetzten siliconhaltigen Polyurethanschaumstoffs sowie durch die gleichmäßige Druckverteilung. Bei der Anwendung des offenzelligen, quervernetzten siliconhaltigen Polyurethanschaumstoffs bei der Behandlung einer durch eine Hauttransplantation entstandenen Wunde kann das Transplantat ("Skin-Graft") ausreichend fixiert werden und gleichzeitig können schädliche Scherkräfte vermieden werden.

Beschrieben wird ein Verfahren zur Unterdrucktherapie von Wunden, umfassend die Schritte
a) Bereitstellen einer Vorrichtung nach einem der Ansprüche 1 bis 12 oder eines Sets nach Anspruch 13,
b) Anlegen des Unterdruckverbandes an der Wunde,
c) Herstellung eines Unterdruckes von höchstens 250 mg Hg, bevorzugt höchstens 175 mm Hg im Wundraum für mindestens 30 Minuten und höchstens 7 Tage, bevorzugt für mindestens 1 Tag und höchstens 6 Tage.

### Figuren

Nachstehend wird die erfindungsgemäße Vorrichtung zur Unterdrucktherapie von Wunden anhand von Zeichnungen näher erläutert. Die Erfindung soll jedoch nicht auf die in den Zeichnungen oder in der Beschreibung der Zeichnung dargestellten Ausgestaltungen reduziert verstanden werden. Vielmehr umfasst die erfindungsgemäße Vorrichtung auch Kombinationen der Einzelmerkmale der alternativen Formen.
- **Figur 1:**: schematischer Aufbau der erfindungsgemäßen Vorrichtung (Seitenansicht)
- **Figur 2:**: schematischer Aufbau einer weiteren Ausführungsform mit Wundkontaktschicht
- **Figur 3:**: schematischer Aufbau einer weiteren Ausführungsform mit Druckverteilungsschicht

### Figurenlegende

1 Wundumgebung (d.h. in der Regel unversehrte Haut)
2 Luftundurchlässiges Abdeckmaterial (a)
3 Offenzelliger Schaumstoff auf Basis des offenzelligen, quervernetzten siliconhaltigen Polyurethanschaumstoffs
4 Unterdruck-Anschlussstück (Port)
5 Unterdruck-Verbindungsleitung
6 Sammelgefäß
7 Unterdruckeinheit
8 Wundgrund
9 Druckverteilungsschicht
10 Wundkontaktschicht

### Beschreibung der Figuren

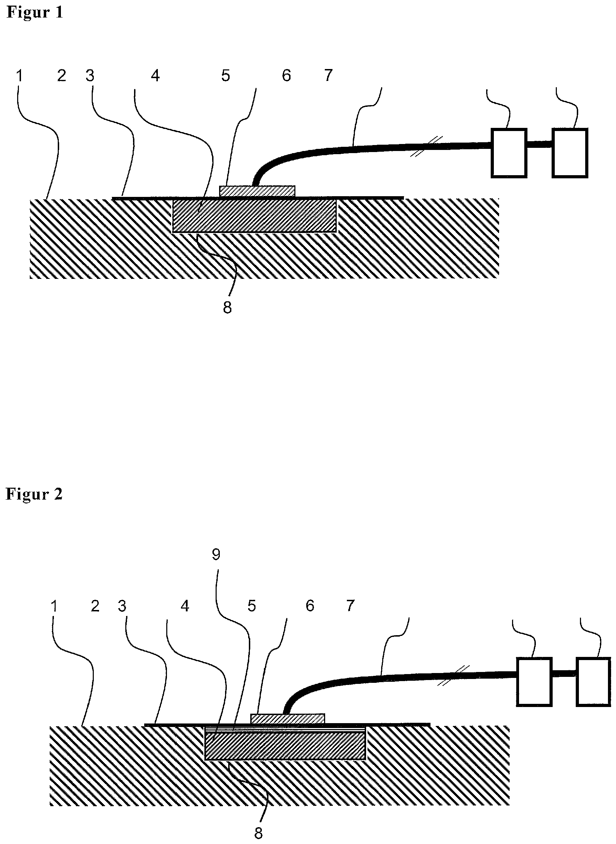

In **Figur 1** wird der schematische Aufbau der erfindungsgemäßen Vorrichtung in der Seitenansicht dargestellt. Die Vorrichtung umfasst ein luftundurchlässiges Abdeckmaterial (2), ein Mittel (4-5) zur funktionellen Verbindung des Wundraums mit einer außerhalb des Abdeckmaterials befindlichen Unterdruckquelle (7), sowie dem offenzelligen, quervernetzten siliconhaltigen Polyurethanschaumstoff (3). Das Abdeckmaterial (2) ist im Bereich der Wundumgebung (1), welche üblicherweise unversehrte Haut aufweist, befestigt. Die Größe des Abdeckmaterials sollte so bemessen sein, dass das Abdeckmaterial außerhalb des Wundbereichs im Bereich der Wundumgebung (1) befestigt werden kann. Das Abdeckmaterial (2) kann in verschiedenen Abmessungen und Formen vorliegen, beispielsweise kreisförmig, oval oder rechteckig. Es kann auch in einer an die Wunde angepassten, unregelmäßigen Form vorliegen. Bei dem Abdeckmaterial (2) kann es sich um ein undurchsichtiges Material, um ein teilweise durchsichtiges Material oder um ein vollständig durchsichtiges Material handeln. Das Abdeckmaterial (2) wird üblicherweise im Bereich der Wundumgebung (1) befestigt und luftdicht abgedichtet. Dies kann beispielsweise dadurch erfolgen, dass das Abdeckmaterial (2) einen Kleberand aufweist. Der Kleberand sollte möglichst bis zum Anlegen des Verbandes durch Schutzstreifen geschützt sein. Alternativ kann eine klebende Substanz entweder auf den Rand des Abdeckmaterials (2) und/oder auf die intakte Haut im Bereich der Wundumgebung aufgebracht werden. Dies hat den Vorteil, dass eine Anpassung des Abdeckmaterials an die Form und Größe der Wunde leichter möglich ist. Eine Befestigung und luftdichte Abdichtung der Vorrichtung kann aber auch durch die Verwendung von Klebestreifen oder einer Klebemasse erreicht werden. Das Unterdruckanschlussstück (4) ist bei der hier gezeigten bevorzugten Ausführungsform auf der von der Wunde weg weisenden Außenseite des luftundurchlässigen Abdeckmaterials (2) angebracht. Um den Wundraum mit einer außerhalb des Abdeckmaterials befindlichen Unterdruckeinheit (7) funktionell zu verbinden, muss sich bei dieser Anordnung im Bereich des Unterdruckanschlussstücks (4) eine oder mehrere durch das Abdeckmaterial (2) hindurchgehende Öffnung(en) befinden. Weiterhin ist eine luftdichte Abdichtung zu gewährleisten. Eine solche Abdichtung kann beispielsweise hergestellt werden, indem auf der von der Wunde weg weisenden Oberseite des Ports eine Folie (in Figur 1 nicht gezeigt) aufgebracht wird, die mit dem Abdeckmaterial (2) verklebt wird. Das Anlegen des Verbandes kann erleichtert werden, wenn ein Port verwendet wird, bei dem ein geeignetes Befestigungs- und Abdichtmittel zum Befestigen des Ports auf dem Abdeckmaterial bereits vorhanden ist. Dies ist beispielsweise bei dem im Handel erhältlichen VivanoTec®- Port der Firma des Anmelders Paul Hartmann AG (Deutschland) der Fall.

**Figur 1** zeigt weiterhin den offenzelligen, quervernetzten siliconhaltigen Polyurethanschaumstoff (3). Beim Anlegen des Verbandes sollte dieser so in den Wundraum eingelegt werden, dass möglichst wenige Hohlräume entstehen und ein guter Kontakt mit dem Wundgrund (8) gewährleistet ist
Der Einsatz einer Wundkontaktschicht (10) in der erfindungsgemäßen Vorrichtung wird in **Figur 2** exemplarisch in der Seitenansicht dargestellt. Hierbei handelt sich um eine bevorzugte Ausführungsform der erfindungsgemäßen Vorrichtung. Die Wundkontaktschicht (10) sollte so beschaffen sein, dass das Wundexsudat die Wundkontaktschicht (10) passieren kann. Es ist auch möglich mehrere Wundkontaktschichten zu verwenden. Dabei können auch unterschiedliche Wundkontaktschichten miteinander kombiniert werden.

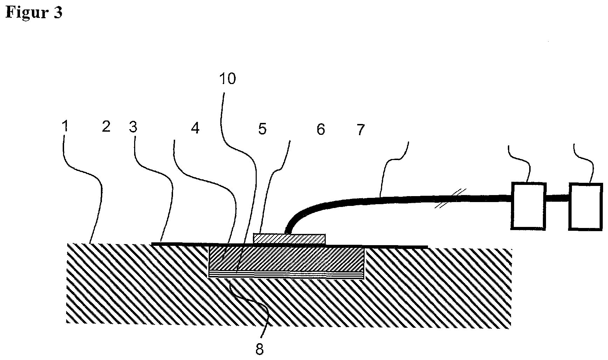

**Figur 3** zeigt den schematischen Aufbau einer weiteren bevorzugten Ausführungsform der erfindungsgemäßen Vorrichtung in der Seitenansicht. Diese Ausführungsform zeichnet sich durch die Anwesenheit einer zusätzlichen Druckverteilungsschicht (9) aus. Es ist auch möglich mehrere Druckverteilungsschichten zu verwenden, wobei unterschiedliche Druckverteilungsschichten miteinander kombiniert werden können. Hervorzuheben ist, dass auch eine Kombination der in Figur 2 gezeigten Wundkontaktschicht mit der in Figur 3 gezeigten zusätzlichen Druckverteilungsschicht von der Erfindung umfasst ist und eine besonders bevorzugte Ausführungsform darstellt.

Die Druckverteilungsschicht (9) kann so ausgebildet sein, dass Flüssigkeit wie Wundexsudat durch sie hindurch geleitet wird. Dazu kann die Druckverteilungsschicht geeignete Kanäle oder Öffnungen enthalten. Alternativ kann sie aus einem Material bestehen, welches die Durchleitung von Wundexsudat ohne weitere Vorkehrungen zulässt.

Die zusätzliche Druckverteilungsschicht (9) kann so ausgestaltet sein, dass sie auf das Mittel zur funktionellen Verbindung des Wundraums mit einer außerhalb des Abdeckmaterials befindlichen Unterdruckquelle abgestimmt ist. Eine solche Anpassung könnte beispielsweise darin bestehen, dass die Druckverteilungsschicht(en) einen Hohlraum zur Aufnahme des Schlauchendes oder des Ports bereitstellt. Andere Anpassungen sind denkbar.

Die zusätzliche Druckverteilungsschicht kann mit dem offenzelligen Schaumstoff (c) haftend oder nicht-haftend verbunden sein.

## Patentansprüche

1. Vorrichtung zur Unterdrucktherapie von Wunden umfassend,
(a) ein Abdeckmaterial zum luftdichten Verschließen der Wunde und der Wundumgebung;
(b) ein Mittel geeignet zur Herstellung von Unterdruck im Wundraum; und
(c) einen offenzelligen, quervernetzten siliconhaltigen Polyurethanschaumstoff als Wundauflage,
wobei der siliconhaltige Polyurethanschaumstoff (c) erhältlich ist durch Umsetzung einer härtbaren Mischung umfassend die Komponenten
(i) Polyisocyanat
(ii) Siloxan der Formel (A) 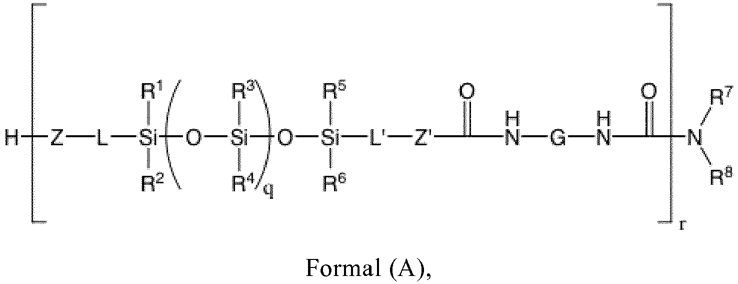 wobei
R¹, R², R³, R⁴, R⁵ und R⁶ unabhängig von einander Wasserstoff, ein substituierter oder ein unsubstituierter aliphatischer Kohlenwasserstoffrest mit 1 bis 8 C-Atomen oder ein substituierter oder ein unsubstituierter aromatischer Kohlenwasserstoffrest mit 6 bis 12 C-Atomen sind,
R⁷ und R⁸ unabhängig von einander Wasserstoff, ein substituierter oder ein unsubstituierter aliphatischer Kohlenwasserstoffrest mit 1 bis 8 C-Atomen oder ein substituierter oder ein unsubstituierter aromatischer Kohlenwasserstoffrest mit 6 bis 12 C-Atomen sind, unter der Voraussetzung, dass mindestens einer der Reste R⁷ und R⁸ mindestens eine Hydroxyl- oder Thiolgruppe aufweist,
Z und Z' unabhängig voneinander Sauerstoff (O) oder Schwefel (S) sind,
L und L' unabhängig von einander ein substituierter oder ein unsubstituierter aliphatischer Kohlenwasserstoffrest mit 1 bis 6 C-Atomen, wobei eines des C-Atome davon durch Sauerstoff (O) ersetzt sein kann, sodass eine Etherbindung vorliegt, oder ein substituierter oder unsubstituierter aromatischer Kohlenwasserstoffrest mit 6 bis 12 C-Atomen ist, welcher ein Heteroatom enthalten kann,
G ein substituierter oder ein unsubstituierter aliphatischer Kohlenwasserstoffrest mit 1 bis 6 C-Atomen, oder ein substituierter oder unsubstituierter aromatischer Kohlenwasserstoffrest mit 6 bis 12 C-Atomen ist, welcher ein Heteroatom enthalten kann,
q eine ganze Zahl von 1 bis 100 ist,
r eine ganze Zahl von 1 bis 10 ist.

2. Vorrichtung zur Unterdrucktherapie von Wunden nach Anspruch 1, wobei die Komponente (i) 2 bis 8 Isocyanatogruppen enthält.

3. Vorrichtung zur Unterdrucktherapie von Wunden nach Anspruch 1 oder 2, wobei in Komponente (ii) R¹, R², R³, R⁴, R⁵ und R⁶ identisch sind.

4. Vorrichtung zur Unterdrucktherapie von Wunden nach Anspruch 3, wobei in Komponente (ii) R¹, R², R³, R⁴, R⁵ und R⁶ Methyl sind.

5. Vorrichtung zur Unterdrucktherapie von einem der Ansprüche 1 bis 4, wobei R⁷ und R⁸ jeweils eine Hydroxyalkylgruppe mit 1 bis 4 C-Atomen sind.

6. Vorrichtung zur Unterdrucktherapie von einem der Ansprüche 1 bis 5, wobei Z und Z' jeweils Sauerstoff (O) sind.

7. Vorrichtung zur Unterdrucktherapie von einem der Ansprüche 1 bis 6, wobei L und L' unabhängig von -CH₂- Methylen oder -CH₂-O-CH₂-CH₂- (Ethoxymethylen) sind.

8. Vorrichtung zur Unterdrucktherapie von einem der Ansprüche 1 bis 7, wobei G 2,4-Toluolylen, 2,6-Toluolylen, 1,3-bis-(2-Propylen)-phenylen, oder Methylen-bis-phenylen ist.

9. Vorrichtung zur Unterdrucktherapie von Wunden nach einem der vorangehenden Ansprüche, wobei der Schaumstoff eine Zugfestigkeit von 100 kPa bis 10 MPa, gemessen gemäß DIN 53571 und/oder eine Bruchdehnung von 100 % bis 350 %, jeweils gemessen gemäß DIN 53571, aufweist.

10. Vorrichtung zur Unterdrucktherapie von Wunden nach einem der vorangehenden Ansprüche, wobei der Schaumstoff (c) eine Luftdurchlässigkeit von 1000 bis 8000 l/(m²sec), gemessen gemäß DIN EN ISO 9237, aufweist.

11. Vorrichtung zur Unterdrucktherapie von Wunden nach einem der vorangehenden Ansprüche, wobei Schaumstoff (c) eine Rohdichte zwischen 0,12 und 0,30 kg/m³ aufweist, gemessen gemäß DIN EN ISO 845.

12. Vorrichtung zur Unterdrucktherapie von Wunden nach einem der vorangehenden Ansprüche, weiterhin umfassend eine Wundkontaktschicht zum Einbringen zwischen Wundoberfläche und Wundauflage (c).

13. Gebrauchsfertiges Set zur Unterdruck-Wundbehandlung umfassend,
a) Abdeckmaterial zum luftdichten Verschließen der Wunde und der Wundumgebung,
b) ein Mittel, geeignet zur Herstellung von Unterdruck im Wundraum, bevorzugt ein Mittel zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind,
c) einen offenzelligen Schaumstoff auf Basis eines quervernetzten, siliconhaltigen Polyurethans, wobei der Schaumstoff als Wundauflage geeignet ist und gebrauchsfertig abgepackt vorliegt und
wobei der siliconhaltige Polyurethanschaumstoff erhältlich ist durch Umsetzung einer härtbaren Mischung umfassend die Komponenten
(i) Polyisocyanat
(ii) Siloxan der Formel (A) 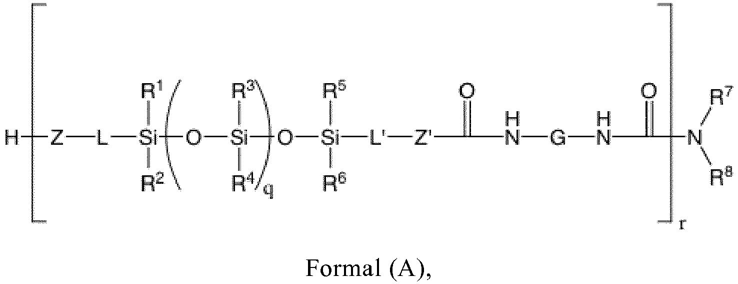 wobei
R¹, R², R³, R⁴, R⁵ und R⁶ unabhängig von einander Wasserstoff, ein substituierter oder ein unsubstituierter aliphatischer Kohlenwasserstoffrest mit 1 bis 8 C-Atomen oder ein substituierter oder ein unsubstituierter aromatischer Kohlenwasserstoffrest mit 6 bis 12 C-Atomen sind,
R⁷ und R⁸ unabhängig von einander Wasserstoff, ein substituierter oder ein unsubstituierter aliphatischer Kohlenwasserstoffrest mit 1 bis 8 C-Atomen oder ein substituierter oder ein unsubstituierter aromatischer Kohlenwasserstoffrest mit 6 bis 12 C-Atomen sind, unter der Voraussetzung, dass mindestens einer der Reste R⁷ und R⁸ mindestens eine Hydroxyl- oder Thiolgruppe aufweist,
Z und Z' unabhängig voneinander Sauerstoff (O) oder Schwefel (S) sind,
L und L' unabhängig von einander ein substituierter oder ein unsubstituierter aliphatischer Kohlenwasserstoffrest mit 1 bis 6 C-Atomen, wobei eines des C-Atome davon durch Sauerstoff (O) ersetzt sein kann, sodass eine Etherbindung vorliegt, oder ein substituierter oder unsubstituierter aromatischer Kohlenwasserstoffrest mit 6 bis 12 C-Atomen ist, welcher ein Heteroatom enthalten kann,
G ein substituierter oder ein unsubstituierter aliphatischer Kohlenwasserstoffrest mit 1 bis 6 C-Atomen, oder ein substituierter oder unsubstituierter aromatischer Kohlenwasserstoffrest mit 6 bis 12 C-Atomen ist, welcher ein Heteroatom enthalten kann,
q eine ganze Zahl von 1 bis 100 ist,
r eine ganze Zahl von 1 bis 10 ist.

## Claims

1. Device for negative pressure wound therapy comprising
(a) a cover material for an airtight enclosure of the wound and its surroundings;
(b) means suitable for generating negative pressure in the wound space; and
(c) an open-cell, crosslinked silicone-containing polyurethane foam as a wound dressing,
wherein the silicone-containing polyurethane foam (c) is obtainable by a reaction of a curable mixture comprising the components
(i) polyisocyanate
(ii) siloxane of Formula (A) 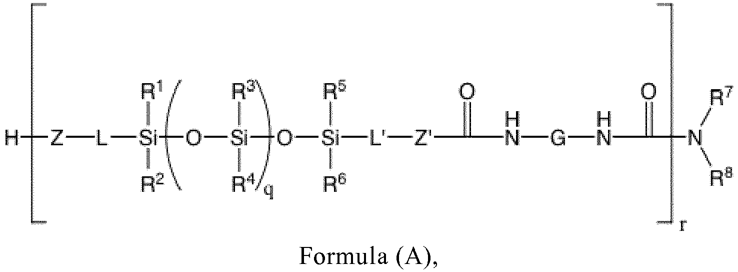 wherein
R¹, R², R³, R⁴, R⁵, and R⁶ are independently hydrogen, a substituted or unsubstituted aliphatic hydrocarbon radical having 1 to 8 carbon atoms or a substituted or unsubstituted aromatic hydrocarbon radical having 6 to 12 carbon atoms,
R⁷ and R⁸ are independently hydrogen, a substituted or unsubstituted aliphatic hydrocarbon radical having 1 to 8 carbon atoms or a substituted or unsubstituted aromatic hydrocarbon radical having 6 to 12 carbon atoms, under the condition that at least one of radicals R⁷ and R⁸ has at least one hydroxyl or thiol group,
Z and Z' are independently oxygen (O) or sulfur (S),
L and L' are independently a substituted or unsubstituted aliphatic hydrocarbon radical having 1 to 6 carbon atoms, wherein one of the carbon atoms thereof may be replaced by oxygen (O) such that there is an ether bond, or a substituted or unsubstituted aromatic hydrocarbon radical having 6 to 12 carbon atoms and optionally containing a heteroatom,
G is a substituted or unsubstituted aliphatic hydrocarbon radical having 1 to 6 carbon atoms or a substituted or unsubstituted aromatic hydrocarbon radical having 6 to 12 carbon atoms, optionally containing a heteroatom,
q is an integer from 1 to 100,
r is an integer from 1 to 10.

2. Device for negative pressure wound therapy according to claim 1, wherein component (i) contains 2 to 8 isocyanate groups.

3. Device for negative pressure wound therapy according to claim 1 or 2, wherein, in component (ii), R¹, R², R³, R⁴, R⁵ and R⁶ are identical.

4. Device for negative pressure wound therapy according to claim 3, wherein, in component (ii), R¹, R², R³, R⁴, R⁵ and R⁶ are methyl.

5. Device for negative pressure wound therapy according to any one of claims 1 to 4, wherein R⁷ and R⁸ are each a hydroxyalkyl group having 1 to 4 carbon atoms.

6. Device for negative pressure wound therapy according to any one of claims 1 to 5, wherein Z and Z' are each oxygen (O).

7. Device for negative pressure wound therapy according to any one of claims 1 to 6, wherein L and L' are independently -CH₂-methylene or -CH₂-O-CH₂-CH₂- (ethoxy methylene).

8. Device for negative pressure wound therapy according to any one of claims 1 to 7, wherein G is 2,4-toluolylene, 2,6-toluolylene, 1,3-bis(2-propylene)-phenylene, or methylene-bis-phenylene.

9. Device for negative pressure wound therapy according to any one of the preceding claims, wherein the foam has a tensile strength of 100 kPa to 10 MPa, measured according to DIN 53571 and/or an elongation at break of 100% to 350%, measured according to DIN 53571, respectively.

10. Device for negative pressure wound therapy according to any one of the preceding claims, wherein the foam (c) has an air permeability of 1000 to 8000 l/(m²sec), measured according to DIN EN ISO 9237.

11. Device for negative pressure wound therapy according to any one of the preceding claims, wherein foam (c) has a gross density between 0.12 and 0.30 kg/m³, measured according to DIN EN ISO 845.

12. Device for negative pressure wound therapy according to any one of the preceding claims, further comprising a wound contact layer to be introduced between the wound surface and the wound dressing (c).

13. Ready-to-use set for negative pressure wound therapy comprising
(a) a cover material for an airtight enclosure of the wound and its surroundings,
(b) means suitable for generating negative pressure in the wound space, preferably means for functionally connecting the wound space to a source of negative pressure which is located outside of the cover material such that negative pressure can be generated in the wound space and that liquids can be aspirated from the wound space,
(c) an open-cell foam based on crosslinked silicone-containing polyurethane, wherein the foam is suitable as a wound dressing and is packaged in a way to be ready for use and
wherein the silicone-containing polyurethane foam is obtainable by a reaction of a curable mixture comprising the components
(i) polyisocyanate
(ii) siloxane of Formula (A) 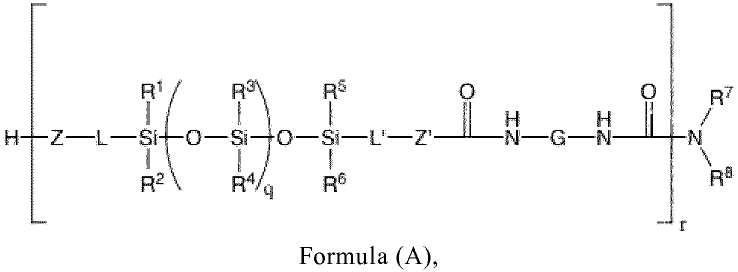 wherein
R¹, R², R³, R⁴, R⁵, and R⁶ are independently hydrogen, a substituted or unsubstituted aliphatic hydrocarbon radical having 1 to 8 carbon atoms or a substituted or unsubstituted aromatic hydrocarbon radical having 6 to 12 carbon atoms,
R⁷ and R⁸ are independently hydrogen, a substituted or unsubstituted aliphatic hydrocarbon radical having 1 to 8 carbon atoms or a substituted or unsubstituted aromatic hydrocarbon radical having 6 to 12 carbon atoms, under the condition that at least one of radicals R⁷ and R⁸ has at least one hydroxyl or thiol group,
Z and Z' are independently oxygen (O) or sulfur (S),
L and L' are independently a substituted or unsubstituted aliphatic hydrocarbon radical having 1 to 6 carbon atoms, wherein one of the carbon atoms thereof may be replaced by oxygen (O) such that there is an ether bond, or a substituted or unsubstituted aromatic hydrocarbon radical having 6 to 12 carbon atoms and optionally containing a heteroatom,
G is a substituted or unsubstituted aliphatic hydrocarbon radical having 1 to 6 carbon atoms or a substituted or unsubstituted aromatic hydrocarbon radical having 6 to 12 carbon atoms, optionally containing a heteroatom,
q is an integer from 1 to 100,
r is an integer from 1 to 10.

## Revendications

1. Dispositif pour la thérapie de plaies par pression négative, le dispositif comprenant
(a) une couverture pour fermer la plaie et l'entourage de la plaie hermétiquement;
(b) des moyens convenant à établir une pression négative dans l'espace de la plaie; et
(c) une mousse de polyuréthane réticulé à alvéoles ouvertes, contenant de la silicone, en tant que pansement,
la mousse de polyuréthane, contenant de la silicone (c), étant susceptible d'être obtenue par une réaction d'un mélange durcissable comprenant les composants
(i) polyisocyanate
(ii) siloxane de formule (A) 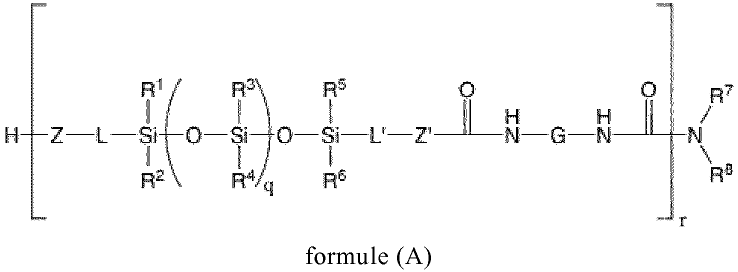 dans laquelle
R¹, R², R³, R⁴, R⁵ et R⁶ sont, indépendamment l'un de l'autre, de l'hydrogène, un radical hydrocarboné aliphatique, substitué ou non-substitué, avec 1 à 8 atomes de carbone ou un radical hydrocarboné aromatique, substitué ou non-substitué, avec 6 à 12 atomes de carbone,
R⁷ et R⁸ sont, indépendamment l'un de l'autre, de l'hydrogène, un radical hydrocarboné aliphatique, substitué ou non-substitué, avec 1 à 8 atomes de carbone ou un radical hydrocarboné aromatique, substitué ou non-substitué, avec 6 à 12 atomes de carbone, à condition qu'au moins un des radicaux R⁷ et R⁸ contienne an moins un groupe hydroxyle ou thiol,
Z et Z' sont, indépendamment l'un de l'autre, de l'oxygène (O) ou du soufre (S), L et L' sont, indépendamment l'un de l'autre, un radical hydrocarboné aliphatique, substitué ou non-substitué, avec 1 à 6 atomes de carbone, où un des atomes de carbone peut être remplacé par de l'oxygène (O) de sorte qu'il y ait une liaison éther, ou un radical hydrocarboné aromatique, substitué ou non-substitué, avec 6 à 12 atomes de carbone lequel peut contenir un hétéroatome,
G est un radical hydrocarboné aliphatique, substitué ou non-substitué avec, 1 à 6 atomes de carbone ou un radical hydrocarboné aromatique, substitué ou non-substitué, avec 6 à 12 atomes de carbone lequel peut contenir un hétéroatome,
q est un entier de 1 à 100,
r est un entier de 1 à 10.

2. Dispositif pour la thérapie de plaies par pression négative selon la revendication 1, le composant (i) contenant 2 à 8 groupes isocyanate.

3. Dispositif pour la thérapie de plaies par pression négative selon la revendication 1 ou 2, R¹, R², R³, R⁴, R⁵ et R⁶ étant identiques dans le composant (ii).

4. Dispositif pour la thérapie de plaies par pression négative selon la revendication 3, R¹, R², R³, R⁴, R⁵ et R⁶ étant méthyle dans le composant (ii).

5. Dispositif pour la thérapie par pression négative selon l'une quelconque des revendications 1 à 4, R⁷ et R⁸ étant chacun un groupe hydroxyle avec 1 à 4 atomes de carbone.

6. Dispositif pour la thérapie par pression négative selon l'une quelconque des revendications 1 à 5, Z et Z' étant chacun de l'oxygène (O).

7. Dispositif pour la thérapie par pression négative selon l'une quelconque des revendications 1 à 6, L et L' étant, indépendamment l'un de l'autre, -CH₂-méthylène ou -CH₂-O-CH₂-CH₂- (éthoxy méthylène).

8. Dispositif pour la thérapie par pression négative selon l'une quelconque des revendications 1 à 7, G étant 2,4-toluolylène, 2,6-toluolylène, 1,3-bis-(2-propylène)-phenylène ou méthylène-bis-phenylène.

9. Dispositif pour la thérapie de plaies par pression négative selon l'une quelconque des revendications précédentes, la mousse ayant une résistance en traction de 100 kPa à 10 MPa, mesurée selon DIN 53571, et/ou un allongement à la rupture de 100 % à 350%, mesurée respectivement selon DIN 53571.

10. Dispositif pour la thérapie de plaies par pression négative selon l'une quelconque des revendications précédentes, la mousse (c) ayant une perméabilité à l'air de 1000 à 8000 l/(m²sec), mesurée selon DIN EN ISO 9237.

11. Dispositif pour la thérapie de plaies par pression négative selon l'une quelconque des revendications précédentes, la mousse (c) ayant une masse volumique apparente entre 0,12 et 0,30 kg/m³, mesurée selon DIN EN ISO 845.

12. Dispositif pour la thérapie de plaies par pression négative selon l'une quelconque des revendications précédentes, comprenant en outre une couche en contact avec la plaie destinée à être introduite entre la surface de la plaie et le pansement (c).

13. Kit prêt à l'emploi pour la thérapie de plaies par pression négative comprenant
a) couverture pour fermer la plaie et l'entourage de la plaie hermétiquement,
(b) un moyen convenant à établir une pression négative dans l'espace de la plaie, de préférence un moyen destinée à relier fonctionnellement l'espace de la plaie avec une source de pression négative qui est située à l'extérieur de la couverture en sorte qu'une pression négative puisse être établie dans l'espace de la plaie et que des liquides puissent être aspirées de l'espace de la plaie,
(c) une mousse à alvéoles ouvertes à base d'un polyuréthane réticulé contenant de la silicone, la mousse convenant à être utilisée en tant que pansement et étant préemballée de manière prête à l'emploi et
la mousse de polyuréthane, contenant de la silicone, étant susceptible d'être obtenue par une réaction d'un mélange durcissable comprenant les composants
(i) polyisocyanate
(ii) siloxane de formule (A) 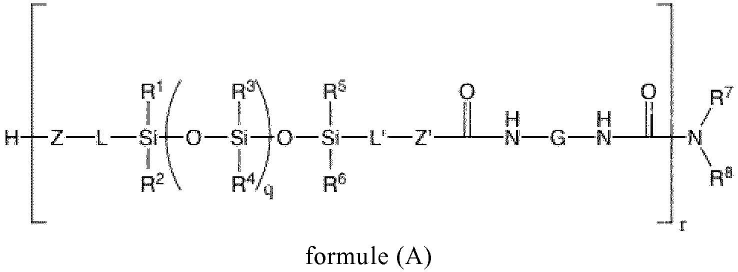 dans laquelle
R¹, R², R³, R⁴, R⁵ et R⁶ sont, indépendamment l'un de l'autre, de'l hydrogène, un radical hydrocarboné aliphatique, substitué ou non-substitué, avec 1 à 8 atomes de carbone ou un radical hydrocarboné aromatique, substitué ou non-substitué, avec 6 à 12 atomes de carbone,
R⁷ et R⁸ sont, indépendamment l'un de l'autre, de l'hydrogène, un radical hydrocarboné aliphatique, substitué ou non-substitué, avec 1 à 8 atomes de carbone ou un radical hydrocarboné aromatique, substitué ou non-substitué, avec 6 à 12 atomes de carbone, à condition qu'au moins un des radicaux R⁷ et R⁸ contienne an moins un groupe hydroxyle ou thiol,
Z et Z' sont, indépendamment l'un de l'autre, de l'oxygène (O) ou du soufre (S), L et L' sont, indépendamment l'un de l'autre, un radical hydrocarboné aliphatique, substitué ou non-substitué avec 1 à 6 atomes de carbone, où un des atomes de carbone peut être remplacé par de l'oxygène (O) de sorte qu'il y ait une liaison éther, ou un radical hydrocarboné aromatique, substitué ou non-substitué, avec 6 à 12 atomes de carbone lequel peut contenir un hétéroatome,
G est un radical hydrocarboné aliphatique, substitué ou non-substitué, avec 1 à 6 atomes de carbone ou un radical hydrocarboné aromatique, substitué ou non-substitué, avec 6 à 12 atomes de carbone lequel peut contenir un hétéroatome,
q est un entier de 1 à 100,
r est un entier de 1 à 10.
